# EUROPEAN PATENT SPECIFICATION

(11) **EP 1 641 931 B1**
(45) Date of publication and mention of the grant of the patent: **19.11.2008**
(21) Application number: 04740419.9
(22) Date of filing: 29.06.2004
(51) Int. Cl.: C12P 7/66, C12N 9/90, C12N 9/12, C12N 9/04, C12R 1/01

(54) **IMPROVED PRODUCTION OF COENZYME Q-10**
VERBESSERTES VERFAHREN ZUR HERSTELLUNG DES COENZYMS Q-10
PRODUCTION AMELIORE DE LA COENZYME Q10

(30) Priority: 08.07.2003 EP 03015367
(43) Date of publication of application: 05.04.2006
(73) Proprietor: DSM IP Assets B.V., 6411 TE Heerlen (NL)
(72) Inventor: BERRY, Alan, Granite Bay, CA 95766 (US); HÜMBELIN, Markus, CH-4058 Basel (CH); LOPEZ-ULIBARRI, Rual, CH-4334 Sisseln (CH)
(74) Representative: Pressner, Dietmar
(86) International application number: PCT/EP2004/007025
(87) International publication number: WO 2005/005650

(56) References cited:
- EP-A- 1 227 155
- WO-A-02/10398
- WO-A-02/26933
- WO-A-02/099095
- WO-A-03/010294
- YOSHIDA H ET AL: "PRODUCTION OF UBIQUINONE-10 USING BACTERIA" JOURNAL OF GENERAL AND APPLIED MICROBIOLOGY, XX, XX, vol. 44, no. 1, 1998, pages 19-26, XP009003546
- BAJ JADWIGA: "Taxonomy of the genus Paracoccus" ACTA MICROBIOLOGICA POLONICA, vol. 49, no. 3-4, 2000, pages 185-200, XP008037036 ISSN: 0137-1320
- HUMBELIN M ET AL: "Genetics of isoprenoid biosynthesis in Paracoccus zeaxanthinifaciens" GENE: AN INTERNATIONAL JOURNAL ON GENES AND GENOMES, ELSEVIER SCIENCE PUBLISHERS, BARKING, GB, vol. 297, no. 1-2, 4 September 2002 (2002-09-04), pages 129-139, XP004388023 ISSN: 0378-1119 cited in the application

## Description

The present invention relates to a method for the production of Coenzyme Q-10 by microorganisms. More particularly, the present invention relates to a method for increased production of Coenzyme Q-10 by transformed microorganisms and the transformants themselves. The transformants are microorganisms of the genus *Rhodobacter,* preferably of the species *R. sphaeroides* which have been transformed with the mevalonate operon from a different microorganism, preferably of the genus *Paracoccus,* more preferably of the species *P*. *zeaxanthinifaciens.*

Coenzyme Q-10 (2,3-dimethoxy-dimethyl-6-decaprenyl-1,4-benzoquinone), also known as ubiquinone-10, is a lipid-soluble benzoquinone having an isoprenoid side chain comprised of ten C-5 isoprenoid units. Coenzyme Q-10 (hereafter abbreviated as CoQ10) is found in microorganisms and plants, as well as in animals. It is the most prevalent form of ubiquinone in humans and most mammals. There is established and growing evidence that CoQ10 is an important factor in the health status of humans and their protection from diseases. The medical and health beneficial effects of CoQ10 have been associated with its two main physiological functions, namely to function as an essential cofactor of the mitochondrial electron transport chain (which is coupled to the synthesis of adenosine triphosphate) and to act as a lipid soluble antioxidant.

The health benefits of CoQ10 have led to increased commercial importance of this compound. CoQ10 can be produced by chemical synthesis or by fermentation using microorganisms. These microorganisms may be natural CoQ10 producers that have been improved for CoQ10 production by genetic engineering, or they may not naturally produce CoQ10 but have been manipulated by genetic engineering to be able to synthesize it.

In bacteria, the quinoid ring of ubiquinones is derived from chorismate, a central intermediate in the biosynthesis of aromatic compounds, while the isoprenoid tail of ubiquinones is derived from the C-5 compound isopentenyl pyrophosphate (IPP). The length of the isoprenoid tail added to the quinoid ring depends of the particular prenyltransferase enzyme that exists in the bacterium. For example, in *Escherichia coli,* octaprenyl pyrophosphate synthase catalyzes the formation of octaprenyl pyrophosphate (C-40) from farnesyl pyrophosphate (FPP, C-15) and five IPP units. Addition of this molecule to the quinoid ring results in formation of ubiquinone-8. In *Paracoccus* and *Rhodobacter* species, decaprenyl pyrophosphate (DPP) synthase catalyzes the formation of DPP (C-50) from FPP (C-15) and seven IPP units. Addition of DPP to the quinoid ring then results in formation of ubiquinone-10 (CoQ10).

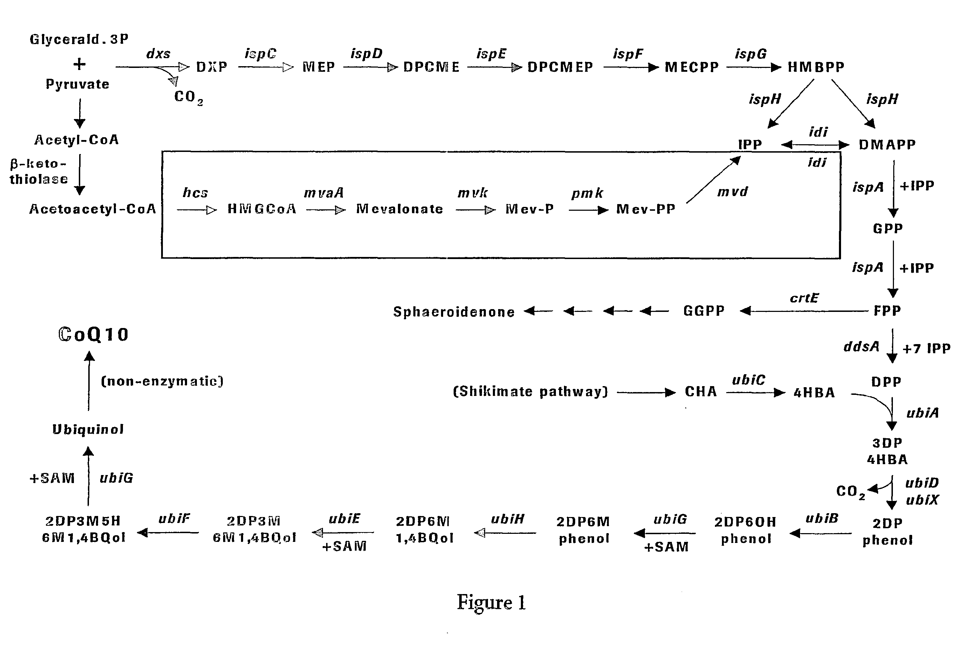

In nature, two different pathways are known for the biosynthesis of IPP (Figure 1). The mevalonate pathway, as its name implies, utilizes mevalonate as a central intermediate and has been well studied in eukaryotes. The mevalonate pathway was thought for many years to be the universal pathway of IPP synthesis in nature. However, in the last decade a second pathway of IPP biosynthesis, called non-mevalonate pathway or the MEP pathway (as it has 2C-methyl-D-erythritol 4-Phosphate as an intermediate) was discovered. The MEP pathway has so far been shown to exist in many eubacteria and in the plastid compartment of higher plants.

US 4,070,244 discloses a method for producing CoQ10 by cultivating certain microorganisms of the genera *Sporidiobolus* and *Oosporidium* in a culture medium containing an assimilable carbon source and a digestible nitrogen source.

Zhu, X. et al. (J. Fermentation & Bioengin., 79, 493-5, 1995) are the first to describe the construction of plasmids containing genes involved in ubiquinone biosynthesis and overexpression of these genes to produce ubiquinone in *E. coli.*

A process for producing CoQ10 by transforming a host microorganism, especially *E*. *coli,* with DNA coding for a protein having decaprenyl diphosphate synthase activity from the genus *Agrobacterium* and culturing the transformant organism is disclosed in EP 1070 759 A1

EP 1 072 683 A1 discloses a process for producing isoprenoid compounds, inter alia ubiquinones, by transformed microorganisms. Among some 70 host species listed *Rhodobacter capsulatus* and *R. sphaeroides* have been mentioned specifically. However, examples only illustrate production of CoQ8 in transformants of *E. coli.*

EP 1 123 979 A1 discloses production of CoQ10 with improved efficiency by transforming *E. coli* with an expression vector comprising DNA coding for decaprenyl diphosphate synthase from a fungal strain of the genus *Saitoella.*

WO 02/099095 A2 finally discloses a method for engineering a bacterium, particularly of the genus *Paracoccus,* to produce an isoprenoid compound, particularly zeaxanthin, by overexpressing genes of the mevalonate pathway.

The gram-negative bacterium *Rhodobacter sphaeroides* is a natural producer of CoQ10, and as such has been attempted to be developed for industrial production of CoQ10. Yoshida et al. (J. Gen. Appl. Microbiol. 44, 19-26, 1998) describe the influence of aeration on CoQ10 production and the isolation of non-recombinant mutants of *R. sphaeroides* that had increased CoQ10 content, and Sakato et al. (Biotechnol. Appl. Biochem. 16, 19-28, 1992) describe methods for increasing CoQ10 production in non-recombinant mutants of *R. sphaeroides* by optimizing the culture (fermentation) conditions. More recently, molecular genetic techniques have been applied to increase production of CoQ10 by *R*. *sphaeroides.*

EP 1 227 155 A1 discloses the increased production of CoQ10 by microorganisms which are capable of producing this compound, inter alia of the genus *Rhodobacter.* The increase in CoQ10 production is achieved by introducing into the microorganisms one or more properties selected from the group consisting of reduced or defective geranylgeranyl transferase activity, strengthened decaprenyldiphosphate synthetase and strengthened p-hydroxybenzoic acid decaprenyl transferase activity.

Based on the presence in *R. sphaeroides* of the *yaeM* gene (now called *ispC*) coding for 1-deoxy-D-xylulose 5-phosphate reductoisomerase and on inspection of the nearly completed genome sequence of *R. sphaeroides,* it appears that this bacterium uses exclusively the MEP pathway for biosynthesis of IPP. Additional evidence supporting the exclusive use of the MEP pathway in *R*. *sphaeroides* is the finding that a closely related species, *Rhodobacter capsulatus,* uses only the MEP pathway for isoprenoid biosynthesis.

WO 02/26933 A1 discloses methods for increasing the production of CoQ10 in *Rhodobacter sphaeroides* by overexpressing a few native and/or heterologous genes coding for some of the enzymes of the MEP pathway. However, the overexpression of these genes resulted in only very modest improvement in CoQ10 production. This is likely due to the fact that the activities of only a few of the enzymes of the MEP pathway were increased, and that overexpression of additional genes is required to significantly increase IPP biosynthesis in *R. sphaeroides.*

As mentioned above, some bacteria use only the mevalonate pathway for biosynthesis of IPP. *Paracoccus zeaxanthinifaciens* is an example of such a bacterium. In *P*. *zeaxanthinifaciens,* the genes coding for the five enzymes of the mevalonate pathway, plus the gene coding for IPP isomerase (see Figure 1), are clustered together in a single transcriptional unit on the chromosome, i.e., an operon called hereinafter the mevalonate operon (Hümbelin et al., Gene 297,129-139, 2002). This unique clustering of all genes required for the conversion of acetoacetyl-CoA to IPP may provide an efficient genetic means to increase IPP production in any bacterium by inserting the cloned mevalonate operon into the cell. This can be achieved not only in bacteria that naturally contain the mevalonate pathway for IPP biosynthesis, but also in bacteria that utilize only the MEP pathway, because the precursor of the mevalonate pathway, acetoacetyl-CoA, is a common metabolite. Acetoacetyl-CoA is produced from two molecules of acetyl-CoA by action of a broad family of enzymes referred to as β-ketothiolases. Thus a supply of acetoacetyl-CoA exists even in bacteria that do not use the mevalonate pathway for IPP biosynthesis.

In an attempt to further increase the production of CoQ10 in microorganisms by methods of genetic engineering of natural CoQ10 producers it has been found that this can be achieved by introducing the cloned mevalonate operon from a microorganism of the genus *Paracoccus* into a microorganism of the genus *Rhodobacter.* While the concept of improving IPP supply and increase of CoQ10 production in *Rhodobacter* by introducing the mevalonate operon from *Paracoccus* might a *prior* seem simple, there is no prior evidence that these genes from *Paracoccus* can be functionally expressed in *Rhodobacter,* nor is there existing evidence that the *Paracoccus* enzymes involved in IPP biosynthesis can function properly in *Rhodobacter.* Moreover, since all available evidence indicates that *Rhodobacter* does not naturally contain the intermediates of the mevalonate pathway, there was the distinct possibility that these non-native compounds would be degraded or otherwise rendered unavailable to the heterologously-expressed *Paracoccus* enzymes.

The present invention relates to a method for increasing CoQ10 production in a microorganism of the genus *Rhodobacter,* particularly of *Rhodobacter sphaeroides*, by introducing into a *Rhodobacter* strain the mevalonate operon of a microorganism of the genus *Paracoccus,* particularly of *Paracoccus zeaxanthinifaciens* and cultivating the transformant. Thus, the present invention provides a process for CoQ10 production comprising introducing a mevalonate operon of a microorganism belonging to the genus *Paracoccus,* particularly of *Paracoccus zeaxanthinifaciens,* into a microorganism belonging to the genus *Rhodobacter,* particularly of *Rhodobacter sphaeroides,* and cultivating the modified *Rhodobacter* strain, whereby the process leads to an increased CoQ10 production.

In other words the present invention relates to a process for producing CoQ10 which comprises culturing, in a medium, a microorganism of the genus *Rhodobacter* into which the mevalonate operon of a microorganism of the genus *Paracoccus* has been introduced, allowing CoQ10 to form and accumulate in the culture and recovering CoQ10 therefrom.

The present invention also relates to a microorganism of the genus *Rhodobacter,* particularly *Rhodobacter sphaeroides,* containing the mevalonate operon of a microorganism of the genus *Paracoccus,* particularly of *Paracoccus zeaxanthinifaciens.*

The present invention also relates to the use of the mevalonate operon of a microorganism of the genus *Paracoccus,* particularly of *Paracoccus zeaxanthinifaciens,* in a method or process for increasing CoQ10 production in a microorganism of the genus *Rhodobacter,* particularly *Rhodobacter sphaeroides.*

The present invention is based on the finding that by introducing the mevalonate operon of a microorganism of the genus *Paracoccus* into a host organism of the genus *Rhodobacter* and cultivating the transformed host the latter is capable of expressing CoQ10 in higher yields.

All process steps which have to be carried out to perform the present invention in practice are conventional process steps with which a person skilled in the art is familiar and which need no specific explanation.

The mevalonate operon to be used in the present invention consists of a DNA sequence of 9066 nucleotides and contains apart from the regulatory sequence (operator) the structural genes coding for the following enzymes necessary for the transformation of acetoacetyl-CoA into DMAPP in the following order: MvaA (hydroxymethylglutaryl-CoA reductase), Idi (isopentenyl diphosphate isomerase), Hcs (hydroxymethylglutaryl-CoA synthase), Mvk (mevalonate kinase), Pmk (phosphomevalonate kinase), and Mvd (diphosphomevalonate decarboxylase). The DNA sequence of this operon is disclosed in WO 02/099095 (see, e.g., SEQ ID NO: 42). This sequence is derived from *Paracoccus* sp, R114 which is a zeaxanthin-producing strain (*P. zeaxanthinifaciens*) and has been deposited under the terms of the Budapest Treaty with ATCC under the Patent Deposit Designation PTA-3335 on April 24, 2001. It has to be noted that the order of the structural genes in the operon differs from the order in which the enzymes they encode catalyze the consecutive metabolic pathway reactions.

The term mevalonate operon of *Paracoccus* or of a microorganism of the genus *Paracoccus* does not mean that the operon which is used in the present invention is actually isolated or derived from a *Paracoccus* organism but rather that it is identical to a mevalonate operon existing in a *Paracoccus* organism. It may, therefore, be isolated completely from *Paracoccus,* totally synthesized or partially isolated and synthesized. Allelic variations and mutants of the DNA sequence of the operon which are fully functional are also comprised by the above term.

With respect to other *Paracoccus* species and strains and the taxonomic reclassification of *Flavobacterium* sp. as *Paracoccus* reference is made to WO 02/099095, pages 47ff. It is understood that the microorganisms " *Paracoccus zeaxanthinifaciens* " and *" Rhodobacter sphaeroides*" also include synonyms or basonyms of such species having the same physicochemical properties, as defined by the International Code of Nomenclature of Prokaryotes.

The term "introducing into" is used in the present specification and claims in connection with the transformation of a microorganism or host organism of the genus *Rhodobacter* to comprise any method well-known to a person skilled in the art which can be used to efficiently bring genetic material, in particular the mevalonate operon, into the host organism, i.e. in a way that it is expressed by the organism. Introduction can be effected, e.g. by vectors, preferably expression plasmids or by integration into the host's genome in accordance with standard methods.

While the preferred host organism for the improved production of CoQ10 in accordance with the present invention is *Rhodobacter sphaeroides* other species of the genus *Rhodobacter* capable of CoQ10 production may as well be used as hosts, e.g., *R*. *adriaticus, R. capsulatus, R. sulfidophilus,* and *R*. *veldkampii.* All host microorganisms may already represent mutants of wild type strains or represent genetically manipulated strains which are characterized by an improved CoQ10 productivity when compared with wild type strain CoQ10 productivity.

After successful introduction of the mevalonate operon into the host microorganism the transformed host is cultivated in accordance with known methods, viz. in a medium containing carbon and nitrogen sources, inorganic salts, etc., which can be assimilated by the host and under temperature, pH and aeration conditions suitable for efficient growing and expression of the desired product, CoQ10.

Isolation from the fermentation broth and/or the transformant, i.e. the microorganism in which the mevalonate operon of a microorganism belonging to the genus *Paracoccus* has been introduced, and, if desired, purification of the CoQ10 obtained including its compounding for human or animal usage can be effected in accordance with methods well-known in the art. For use in animal health and nutrition, however, no specific purification may be necessary. In this case CoQ10 together with the biomass and/or other components of the fermentation broth maybe further processed to yield a commercially attractive product.

Figure 1 represents the pathway for CoQ10 biosynthesis in *R*. *sphaeroides,* which uses the MEP pathway for IPP formation. The boxed region indicates the reaction sequence that comprises the mevalonate pathway (leading to formation of IPP), plus the IPP isomerase step. The mevalonate pathway does not naturally occur in *R*. *sphaeroides.* In *P*. *zeaxanthinifaciens*, the genes coding for the five enzymes of the mevalonate pathway plus IPP isomerase form an operon, called hereinafter the mevalonate operon.

The following Example illustrates the invention without restricting it in any way.

### Example

This example shows the improvement in CoQ10 production in *R*. *sphaeroides* DSM 158 upon introduction of the cloned mevalonate operon from *Paracoccus zeaxanthinifaciens.*

### Bacteria and culture conditions.

*Rhodobacter sphaeroides* strain DSM 158 (obtained from the Deutsche Sammlung von Mikroorganismen und Zellkulturen GmbH) was used as the base host for construction of recombinant strains having improved production of CoQ10. All *R. sphaeroides* strains were grown at 28°C in medium RS100. The composition and preparation of medium RS 100 is summarized in Table 1. Fifty mg/l kanamycin was added to the medium for growth of recombinant strains. *E*. *coli* strains were grown at 37°C in LB medium (Becton Dickinson, Sparks, MD, USA). For maintenance of plasmids in recombinant *E*. *coli* strains, ampicillin (100 mg/l) and/or kanamycin (25-50 mg/l, depending on the plasmid) were added to the culture medium. Liquid cultures of *E*. *coli* and *R*. *sphaeroides* were routinely grown aerobically in a rotary shaker at 200 rpm. When solid media were required, agar (1.5% final concentration) was added.

**Table 1 Composition and preparation of medium RS100**

| **Medium RS100** | |
|---|---|
| Component | Amount (per liter distilled water) |
| 1. Yeast extract | 10 g |
| 2. Peptone | 10 g |
| 3. NaCl | 0.5 g |
| 4. MgSO₄·7H₂O | 0.5 g |
| 5. D-glucose monohydrate | 33 g |
| Components 1-5 are mixed together, the final volume is adjusted to 1 liter, and the pH is adjusted to 7.4 with 0.5 M NaOH. The resulting base medium is then sterilized by filtration through a 0.22 micron membrane. Two ml each of sterile Microelements solution and sterile CaFe solution (see below) are added to give the final medium RS100. For solid medium, components 1-5, plus 15 g agar, are first mixed together and autoclaved. After the medium is cooled down to about 60°C, the sterile microelements and CaFe solutions (2 ml of each) are added and the molten medium is mixed well and dispensed into sterile petri plates. | |
| | |

| **Microelements solution** | |
|---|---|
| Component | Amount per liter distilled water |
| (NH₄)₂Fe(SO₄)₂-6H₂O | 80 g |
| ZnSO₄·7H₂O | 6 g |
| MnSO₄·H₂O | 2 g |
| NiSO₄·6H₂O | 0.2 g |
| EDTA | 6 g |
| - Sterilize by filtration through a 0.22 micron membrane, store at 4°C. | |
| | |
| | |

| **CaFe solution** | |
|---|---|
| Component | Amount per liter distilled water |
| CaCl₂·2H₂O | 75 g |
| FeCl₃·6H₂O | 5 g |
| HCl (37%) | 3.75 ml |
| - Sterilize by filtration through a 0.22 micron membrane, store at 4°C. | |

### Construction of plasmids pBBR-K-Nde and pBBR-K-mev-opR114.

The construction of plasmids pBBR-K-Nde (empty plasmid) and pBBR-K-mev-op-up-4 (plasmid containing the first 4 genes of the mevalonate operon) are described in detail in Examples 6 (page 91, lines 12 - 17) and 13 (page 105, line 10 - page 106, line 8), respectively of WO 02/099095.

PCR primer hcs-5326 (SEQ ID NO: 1) corresponds to the sequence of the *P. zeaxanthinifaciens hcs* gene (starting at codon 214 and ending with the second nucleotide of codon 220), while PCR primer mvd-9000 (SEQ ID NO: 2) corresponds to the reverse complement of the sequence of nucleotides 104 through 123 after the stop codon of the *P. zeaxanthinifaciens mvd* gene (WO 02/099095, see SEQ ID Nos. 46 and 52, respectively). Plasmid TOPO-pCR2.1-mev-op-d-3wt was constructed by cloning the PCR fragment obtained with primers hcs-5326 and mvd-9000 (using *P*. *zeaxanthinifaciens* ATCC 21588 genomic DNA as template) in pCR2.1-TOPO. This plasmid thus contains the downstream half of the mevalonate operon including the 3' end *of hcs* and the last three genes, *mvk, pmk* and *mvd*. Plasmids pBBR-K-mev-op-up-4 and TOPO-pCR2.1-mev-op-d-3wt were digested with the restriction endonucleases *Sac*I and *Nde*I and the shorter fragment from the latter digest was ligated with the larger fragment from pBBR-K-mev-op-up-4. The resulting plasmid, pBBR-K-mev-op-wt, contains the complete mevalonate operon from *P. zeaxanthinifaciens* ATCC 21588 including its (putative) promoter. The only difference between the mevalonate operon sequences of the *P*. *zeaxanthinifaciens* strains ATCC 21588 (wild-type) and R114 is a mutation in mvk, resulting in a change of residue 265 from alanine to valine (A265V). Because *P*. *zeaxanthinifaciens* ATCC 21588 genomic DNA was used as PCR template for the construction of plasmid TOPO-pCR2.1-mev-op-d-3wt, it contains the wild-type *mvk* gene. Introduction of the mutation in *mvk* (codon 265 changed from GCC to GTC) by site-directed mutagenesis resulted in plasmid pBBR-K-mev-op-R114.

### Transformation procedures for R. sphaeroides.

Transformation of *E*. *coli* S17-1 with plasmids for cloning and subsequent transfer of plasmids from S17-1 to *R. sphaeroides* DSM 158 by conjugation were performed using standard procedures (Nishimura *et al.,* Nucl. Acids Res. 18, 6169, 1990; Simon et al., Bio/Technology 1983, 784 - 91). A spontaneous rifampicin resistant mutant of *R. sphaeroides* DSM 158 was first isolated by growing DSM 158 in RS100 liquid medium supplemented with 100 mg/l rifampicin, plating the cells on RS100 plates containing 100 mg/l rifampicin, and isolating a single colony. For the conjugation, one milliliter aliquots of cultures of the recipient cells (rifampicin-resistant *R. sphaeroides* DSM 158) grown in RS100 medium containing 100 mg/l rifampicin) and the donor cells (*E. coli* S17-1 carrying the plasmid to be transferred, grown in LB broth containing 50 mg/l kanamycin) were pelleted by centrifugation. The supernatant was discarded, and the cells were washed twice with fresh RS100 medium to remove antibiotics. Each pellet was then resuspended in 1 ml of fresh RS100 medium. Fifty microliters of donor cells and 0.45 ml of recipient cells were mixed, pelleted by centrifugation, resuspended in 0.03 ml of fresh RS100 medium and spotted onto an RS100 plate. After overnight incubation at 28°C the cells were harvested with an inoculating loop and resuspended in 0.3 ml of RS100 medium. Dilutions of this suspension were spread onto RS100 plates containing 100 mg/l rifampicin and 50 mg/l kanamycin and incubated at 28°C. Colonies (putative transformed cells of *R*. *sphaeroides* DSM 158) were picked from the plates, grown in liquid RS 100 medium containing 50 mg/l kanamycin and the presence of the plasmid was tested by PCR using appropriate primers. Positive clones were streaked onto RS100 plates containing 50 mg/l kanamycin to obtain single colonies. One single colony from each clone was again grown in liquid RS100 medium containing 50 mg/l kanamycin, and the presence of the expected plasmid was confirmed by PCR. The final transformed strains of *R. sphaeroides* DSM 158 were preserved by adding glycerol to the culture (15% v/v) and freezing at -80°C.

### Culture conditions for evaluating CoO10 production in R. sphaeroides.

Medium RS100 was used for all experiments with *R*. *sphaeroides* (50 mg/l kanamycin was added for growth of recombinant *R*. *sphaeroides* strains). Twenty five-milliliter cultures were grown at 28°C in 250-ml baffled Erlenmeyer flasks with shaking at 200 rpm. For testing CoQ10 production, frozen glycerolized stock cultures of the *R*. *sphaeroides* strains were used to inoculate 25-ml seed cultures. After growth of the seed cultures for 24-28 hours, suitable volumes of the cultures were used to inoculate the experimental flasks such that the initial optical density at 660 nanometers (OD₆₆₀) was 0.16. Two milliliter samples were taken aseptically at 24 hour intervals. Analyses included growth (measured as OD₆₆₀), pH, glucose in the culture supernatant and CoQ10 and carotenoids (determined by high performance liquid chromotography [HPLC] - see Analytical Methods section below).

### Analytical methods.

Reagents: Acetonitrile, dimethylsulfoxide (DMSO), tetrahydrofuran (THF), tert-butyl methyl ether (TBME) and butylated hydroxytoluene (BHT) were puriss.p.a. or HPLC grade and were obtained from Fluka (Switzerland). CoQ10 was also purchased from Fluka. Methanol (Lichrosolv) was purchased from Merck, Darmstadt. Carotenoid standards were obtained from the Chemistry Research Department, Roche Vitamins Ltd., Switzerland.

Sample preparation and extraction: Four hundred microliters of whole broth were transferred to a disposable 15 ml polypropylene centrifuge tube. Four milliliters of stabilized extraction solution (0.5 g/l BHT in 1:1 (v/v) DMSO/THF) were added and the samples were mixed for 20 minutes in a laboratory shaker (IKA, Germany) to enhance extraction. Finally, the samples were centrifuged and the supernatants transferred to amber glass vials for analysis by HPLC.

HPLC: A reversed phase HPLC method was developed for the simultaneous determination of ubiquinones and their corresponding hydroquinones. The method is able to clearly separate the carotenoids phytoene, spheroidenone, spheroidene and neurosporene from CoQ10. Chromatography was performed using an Agilent 1100 HPLC system equipped with a temperature-controlled autosampler and a diode array detector. The method parameters were as follows:

| | | | | |
|---|---|---|---|---|
| Column | YMC Carotenoid C30 column | | | |
| | 3 micron, steel, 150 mm x 3.0 mm LD. | | | |
| | (YMC, Part No. CT99S031503QT) | | | |
| | | | | |
| Guard column | Security Guard C18 (ODS, Octadecyl) | | | |
| | 4 mm length x 3.0 mm I.D. | | | |
| | (Phenomenex, Part No. AJO-4287) | | | |
| | | | | |
| Typical column pressure | 60 bar at start | | | |
| | | | | |
| Flow rate | 0.5 ml/min | | | |
| | | | | |
| Mobile phase | Mixture of acetonitrile(A):methanol(B):TBME(C) | | | |
| | | | | |

| Gradient profile | Time (min) | %A | %B | %C |
|---|---|---|---|---|
| | 0 | 60 | 15 | 25 |
| | 13 | 60 | 15 | 25 |
| | 20 | 00 | 100 | |
| | 22 | 60 | 15 | 25 |
| | 25 | 60 | 15 | 25 |
| | | | | |
| Post time | 4 min. | | | |
| | | | | |
| Injection volume | 10 µl | | | |
| | | | | |
| Column temperature | 15°C | | | |
| | | | | |
| Detection | Three wavelengths were used for detection of specific | | | |
| | compounds according to Table 2. | | | |

**Table 2. HPLC retention times and wavelengths used.**

| **Compound** | **Wavelength (nm)** | **Retention times (min)** |
|---|---|---|
| Phytoene | 280 | 7.9 |
| Ubiquinol-10 | 210 | 11.3 |
| CoQ10 | 210 | 12.6 |
| Spheroidenone (Z-isomers) | 450 | 10.6, 13.0, 14.6, 18.7 |
| E-Spheroidenone | 450 | 19.1 |
| E-Neurosporene | 450 | 20.4 |
| E-Spheroidene | 450 | 20.6 |

### Calculations: Calculations were based on peak areas.

Selectivity: The selectivity of the method was verified by injecting standard solutions of the relevant reference compounds. The target compounds (CoQ10 and ubiquinol-10) were completely separated and showed no interference.

Linearity and limit of detection: A dilution series of CoQ10 in stabilized extraction solution (see above) was prepared and analyzed. A linear range was found from 5 mg/l to 50 mg/l. The correlation coefficient was 0.9999. The limit of detection for CoQ10 by this HPLC method was determined to be 4 mg/l.

Reproducibility: The reproducibility of the method including the extraction procedure was checked. Ten individual sample preparations were compared. The relative standard deviation was determined to be 4%.

### Determination of CoQ10 production by R. sphaeroides strains.

*R. sphaeroides* strains DSM 158, DSM 158/pBBR-K-Nde (empty vector control) and DSM 158/pBBR-K-mev-opR114 were grown in shake flask cultures under the conditions specified above, and CoQ10 production was determined. The results are summarized in Table 3. The values shown are the average of two independent experiments, and within each independent experiment, each strain was tested in duplicate. These results clearly show that the expression of the cloned mevalonate operon from *P*. *zeaxanthinifaciens* significantly improved CoQ10 production in *R. sphaeroides.*

**Table 3**

| | | CoQ10 | |
|---|---|---|---|
| Strain | Time (hr) | mg/l (SD) | Specific Formation¹ (SD) |
| DSM 158 | 24 | 15.76 (0.42) | 0.83 (0.01) |
| DSM 158/pBBR-K-Nde | | 14.44 (1.67) | 0.85 (0.04) |
| DSM 158/pBBR-K-mev-opR114 | | 22.28 (1.24) | 1.25 (0.06) |
| | | | |
| DSM 158 | 48 | 28.85 (0.51) | 1.36 (0.04) |
| DSM 158/pBBR-K-Nde | | 25.99 (0.20) | 1.18 (0.08) |
| DSM 158/pBBR-K-mev-opR114 | | 63.39 (2.06) | 2.28 (0.03) |
| | | | |
| DSM 158 | 72 | 33.12 (0.23) | 2.04 (0.01) |
| DSM 158/pBBR-K-Nde | | 29.71 (0.39) | 1.79 (0.10) |
| DSM 158/pBBR-K-mev-opR114 | | 85.09 (3.42) | 3.80 (0.01) |

| | | | |
|---|---|---|---|
| ¹Specific Formation is expressed as mg CoQ10 produced/mg cell dry mass. SD = standard deviation. | | | |

## Claims

1. A process for CoQ10 production comprising introducing a mevalonate operon of a microorganism belonging to the genus *Paracoccus* into a microorganism belonging to the genus *Rhodobacter* and cultivating the modified *Rhodobacter* strain, wherein the said mevalonate operon contains apart from the regulatory sequence (operator) the structural genes coding for the following enzymes: MvaA (hydroxymethylglutaryl-CoA reductase), Idi (isopentenyl diphosphate isomerase), Hcs (hydroxymethylglutaryl-CoA synthase), Mvk (mevalonate kinase), Pmk (phosphomevalonate kinase), and Mvd (diphosphomevalonate decarboxylase).

2. The process of claim 1, wherein *R. sphaeroides* is used as the CoQ10 producing microorganism.

3. The process of claim 1 or 2 wherein the mevalonate operon of *Paracoccus zeaxanthinifaciens* is introduced into the *Rhodobacter* strain.

4. A process for producing CoQ10 which comprises culturing, in a medium, a microorganism of the genus *Rhodobacter* into which the mevalonate operon of a microorganism of the genus *Paracoccus* has been introduced, allowing CoQ10 to form and accumulate in the culture and recovering CoQ10 therefrom.

5. A microorganism of the genus *Rhodobacter* containing the mevalonate operon of a microorganism of the genus *Paracoccus.*

6. The microorganism of claim 5 which is *Rhodobacte*r *sphaeroides.*

7. The microorganism of claim 5 or 6 containing the mevalonate operon of *Paracoccus zeaxanthinifaciens.*

8. The use of the mevalonate operon of a microorganism of the genus *Paracoccus* in a process as claimed in any one of claims 1 to 4.

9. A method for increasing CoQ10 production in a microorganism of the genus *Rhodobacter* by introducing into a *Rhodobacter* strain the mevalonate operon of a microorganism of the genus *Paracoccus* and cultivating the transformant.

## Patentansprüche

1. Verfahren zur Herstellung von CoQ10, bei dem man ein Mevalonat-Operon eines Mikroorganismus der Gattung *Paracoccus* in einen Mikroorganismus der Gattung *Rhodobacter* einführt und den modifizierten Rhodobacter-Stamm kultiviert, wobei das Mevalonat-Operon abgesehen von der Regulationssequenz (Operator) die für die folgenden Enzyme codierenden Strukturgene enthält: MvaA (Hydroxymethylglutaryl-CoA-Reduktase), Idi (Isopentenyldiphosphatisomerase), Hcs (Hydroxymethylglutaryl-CoA-Synthase), Mvk (Mevalonatkinase), Pmk (Phosphomevalonatkinase) und Mvd (Diphosphomevalonatdecarboxylase).

2. Verfahren nach Anspruch 1, wobei man als CoQ10-produzierenden Mikroorganismus *R. sphaeroides* verwendet.

3. Verfahren nach Anspruch 1 oder 2, wobei das Mevalonat-Operon von *Paracoccus zeaxanthinifaciens* in den *Rhodobacter*-Stamm eingeführt wird.

4. Verfahren zur Herstellung von CoQ10, bei dem man einen Mikroorganismus der Gattung *Rhodobacter*, in den das Mevalonat-Operon eines Mikroorganismus der Gattung *Paracoccus* eingeführt worden ist, in einem Medium kultiviert, CoQ10 bilden und in der Kultur anreichern läßt und CoQ10 aus der Kultur gewinnt.

5. Mikroorganismus der Gattung *Rhodobacter*, der das Mevalonat-Operon eines Mikroorganismus der Gattung *Paracoccus* enthält.

6. Mikroorganismus nach Anspruch 5, wobei es sich um *Rhodobacter sphaeroides* handelt.

7. Mikroorganismus nach Anspruch 5 oder 6, der das Mevalonat-Operon von *Paracoccus zeaxanthinifaciens* enthält.

8. Verwendung des Mevalonat-Operons eines Mikroorganismus der Gattung *Paracoccus* in einem Verfahren nach einem der Ansprüchen 1 bis 4.

9. Verfahren zur Erhöhung der CoQ10-Produktion in einem Mikroorganismus der Gattung *Rhodobacte*r durch Einführen des Mevalonat-Operons eines Mikroorganismus der Gattung *Paracoccus* in einen *Rhodobacter-Stamm* und Kultivieren der Transformante.

## Revendications

1. Procédé de production de CoQ10 consistant à introduire un opéron mévalonate d'un microorganisme appartenant au genre *Paracoccus* dans un microorganisme appartenant au genre *Rhodobacter* et à mettre en culture la souche de *Rhodobacter* modifiée, selon lequel ledit opéron mévalonate contient, outre la séquence régulatrice (opérateur), les gènes structuraux codant pour les enzymes suivantes : MvaA (hydroxyméthylglutaryl-CoA réductase), Idi (isopentényl-diphosphate isomérase), Hcs (hydroxyméthylglutaryl-CoA synthase), Mvk (mévalonate kinase), Pmk (phosphomévalonate kinase) et Mvd (diphosphomévalonate décarboxylase).

2. Procédé selon la revendication 1, selon lequel *R. sphaeroides* est utilisé à titre de microorganisme producteur de CoQ10.

3. Procédé selon la revendication 1 ou 2, selon lequel l'opéron mévalonate de *Paracoccus zeaxanthinifaciens* est introduit dans la souche de *Rhodobacter.*

4. Procédé de production de CoQ10 qui consiste à mettre en culture, dans un milieu, un microorganisme du genre *Rhodobacter* dans lequel l'opéron mévalonate d'un microorganisme du genre *Paracoccus* a été introduit, à laisser le CoQ10 se former et s'accumuler dans la culture et à récupérer le CoQ10 à partir de celle-ci.

5. Microorganisme du genre *Rhodobacter* contenant l'opéron mévalonate d'un microorganisme du genre *Paracoccus*.

6. Microorganisme selon la revendication 5, qui est *Rhodobacter sphaeroides.*

7. Microorganisme selon la revendication 5 ou 6, contenant l'opéron mévalonate de *Paracoccus zeaxanthinifaciens.*

8. Utilisation de l'opéron mévalonate d'un microorganisme du genre *Paracoccus* dans un procédé selon l'une quelconque des revendications 1 à 4.

9. Méthode permettant d'accroître la production de CoQ10 chez un microorganisme du genre *Rhodobacter* par l'introduction dans une souche de *Rhodobacter* de l'opéron mévalonate d'un microorganisme du genre *Paracoccus* et la mise en culture du transformant.


### SEQUENCE LISTING

<110> DSM IP Assets B.V.
<120> Improved production of coenzyme Q-10
<130> Case 21841
<150> EP 03015367.0
   <151> 2003-07-08
<160> 2
<170> PatentIn version 3.2
<210> 1
   <211> 20
   <212> DNA
   <213> Artificial
<220>
   <223> PCR primer hcs-5336
<400> 1 20
   gcgctggtcg aggcgtggaa 20
<210> 2
   <211> 20
   <212> DNA
   <213> Artificial
<220>
   <223> PCR primer mvd-9000
<400> 2 20
   cgtcagcgtg gcgggcaagt 20